(19) Europäisches Patentamt
European Patent Office
Office européen des brevets

(11) **EP 1 339 376 B1**

(12) **FASCICULE DE BREVET EUROPEEN**

(45) Date de publication et mention
de la délivrance du brevet:
**15.07.2009 Bulletin 2009/29**

(21) Numéro de dépôt: **01997284.3**

(22) Date de dépôt: **22.11.2001**

(51) Int Cl.:
*A61K 8/85* *(2006.01)*     *A61K 8/88* *(2006.01)*
*A61Q 1/02* *(2006.01)*     *A61Q 1/08* *(2006.01)*
*A61Q 1/10* *(2006.01)*     *A61Q 3/02* *(2006.01)*

(86) Numéro de dépôt international:
**PCT/FR2001/003695**

(87) Numéro de publication internationale:
**WO 2002/041855 (30.05.2002 Gazette 2002/22)**

(54) **COMPOSITION COSMETIQUE COMPRENANT DES FIBRES INTERFERENTIELLES ET UNE MATIERE COLORANTE**

KOSMETISCHE ZUSAMMENSETZUNG MIT INTERFERENZFASERN UND EINEM FÄRBEMITTEL

COSMETIC COMPOSITION COMPRISING INTERFERENTIAL FIBERS AND A COLOURING MATERIAL

(84) Etats contractants désignés:
**AT BE CH CY DE DK ES FI FR GB GR IE IT LI LU MC NL PT SE TR**

(30) Priorité: **23.11.2000  FR 0015135**

(43) Date de publication de la demande:
**03.09.2003  Bulletin 2003/36**

(73) Titulaire: **L'ORÉAL**
**75008 Paris (FR)**

(72) Inventeurs:
• **BLIN, Xavier**
  **F-75006 Paris (FR)**
• **JAGER LEZER, Nathalie**
  **91370 Verrières le Buisson (FR)**
• **SIMON, Jean-Christophe**
  **64665 Alsbach-Hähnlein (DE)**

(74) Mandataire: **Kromer, Christophe**
**L'OREAL - D.I.P.I.**
**25-29 Quai Aulagnier**
**92600 Asnières (FR)**

(56) Documents cités:
**WO-A1-00/75240**          **GB-A- 1 290 351**
**US-A- 5 370 866**

• **PATENT ABSTRACTS OF JAPAN vol. 009, no. 130 (C-284), 5 juin 1985 (1985-06-05) & JP 60 016910 A (SHISEIDO KK), 28 janvier 1985 (1985-01-28)**

Remarques:
Le dossier contient des informations techniques présentées postérieurement au dépôt de la demande et ne figurant pas dans le présent fascicule.

EP 1 339 376 B1

**Description**

La présente invention se rapporte à une composition cosmétique selon la revendication 1 contenant des fibres interférentielles, et plus spécialement à une composition de maquillage de la peau aussi bien du visage que du corps humain, des lèvres et des phanères comme les ongles, les cils, les sourcils où les cheveux.

La composition selon l'invention peut être une composition de maquillage colorée comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, les produits de maquillage du corps, les produits de maquillage des cheveux (mascara ou laque pour cheveux). La composition peut être utilisée telle quelle pour l'application sur les matières kératiniques ou bien encore être appliquée sur un maquillage déjà déposé sur les matières kératiniques, par exemple pour modifier le maquillage (la composition est appliquée comme produit de surface communément appelée top coat en terminologie anglo-saxonne).

La composition de maquillage peut également être appliquée sur les accessoires de maquillage (support) comme les faux ongles, faux cils, postiches, perruques ou encore sur des pastilles ou patchs adhérents sur la peau ou les lèvres (du type mouches).

Il est connu d'utiliser des fibres dans des produits de maquillage notamment pour leurs effets allongeants dans des mascaras (voir JP-A-571158714), leurs propriétés hydratantes dans des rouges à lèvres (voir le document US-A-5 498407), pour améliorer les contours du rouge à lèvres sur les bords des lèvres (voir le document EP-A-0106762) ou pour remettre en état les ongles cassés (voir FR-A-1529329) ou bien encore dans des produits de soin de la peau pour leur toucher velouté (voir JP-A-7/196440). Les compositions de maquillage contenant des fibres contiennent également des matières colorantes comme les pigments minéraux ou organiques.

Il est également connu du document EP-A-921217 des fibres présentant un effet de couleur par interférences optiques, appelées fibres interférentielles.

Par ailleurs, les compositions de maquillage, comme les vernis à ongles, sont habituellement colorées à l'aide de matières colorantes comme les colorants solubles, les pigments qui sont généralement des oxydes métalliques, tels que les oxydes de fer, ou bien encore à l'aide de nacres tels que les micas recouverts d'oxydes métalliques comme l'oxyde de titane.

Or, les inventeurs ont constaté que les fibres interférentielles mentionnées précédemment peuvent perdre leur effet de couleur particulier lorsqu'elles sont incorporées dans des compositions de maquillage contenant des agents de coloration et en particulier des pigments ; notamment, les inventeurs ont constatés que si la composition comprenait trop de pigments, ces derniers allaient masquer l'effet de couleur particulier des fibres interférentielles.

La but de la présente invention est donc de proposer une composition de maquillage ne présentant pas les inconvénients ci-dessus et présentant un effet de couleur original.

Les inventeurs ont découvert qu'une telle composition peut être obtenue en associant des fibres interférentielles selon la revendication 1 à un agent de coloration additionnel en des quantité particulières. La composition permet d'obtenir un maquillage présentant les effets de couleur des particules interférentielles.

La composition appliquée sur la peau permet d'obtenir un bon camouflage des imperfections de la peau, un maquillage illuminateur de teint. Le maquillage obtenu présente également un effet visuel brillant.

De plus, les inventeurs ont également découvert que ces fibres s'incorporent très facilement dans les compositions cosmétiques et se répartissent dans la composition de façon homogène.

JP 2003-19131 décrit une composition cosmétique contenant des fibres interférentielles à base de poudre de polymére comme par exemple polyéthylène téréphtalate ayant une gamme de L/D entre 3-1 et 100-1.

L'incorporation des fibres interférentielles dans la composition se fait très facilement, aussi bien à froid qu'à chaud sans perdre les propriétés cosmétiques et/ou optiques de la composition. En particulier, la bonne homogénéité des fibres dans la composition ne change pas l'aspect du produit. Il est possible d'incorporer des teneurs importantes en fibres dans la composition sans modifier l'aspect visuel de la composition.

La composition appliquée sur les matières kératiniques présente également comme avantage de former un dépôt présentant une sensation de velours au toucher dû à la dispersion homogène des fibres dans la composition et dans le dépôt formé après l'application. La composition apporte donc un toucher différent des touchers lisses, craquelés ou granuleux, satisfaisant ainsi les consommateurs à la recherche de nouveauté.

En outre, les fibres interférentielles apportent des propriétés de renfort mécanique de la composition et du dépôt formé après application sur les matières kératiniques, en particulier lorsque le dépôt comprend un polymère filmogène. En particulier, la composition forme un dépôt présentant de bonnes propriétés de résistance mécanique : le dépôt est bien résistant aux frottements, aux chocs, aux rayures. Le dépôt est également bien résistant à l'eau (notamment lors de la baignade ou de la douche), de la pluie, des larmes, de la sueur, du sébum. Les fibres apportent ainsi une meilleure tenue du dépôt sur les matières kératiniques.

De façon plus précise, l'invention a pour objet une composition comprenant, dans un milieu cosmétiquement acceptable, des fibres interférentielles présentant un effet de couleur et une matière colorante additionnelle, ladite matière colorante étant en quantité suffisante pour ne pas masquer l'effet de couleur desdites particules.

L'invention a également pour objet un procédé cosmétique de maquillage des matières kératiniques comprenant l'application sur les matières kératiniques d'une composition telle que définie précédemment.

L'invention a également pour objet l'utilisation de fibres interférentielles présentant un effet de couleur et d'un agent de coloration additionnel, ledit agent de coloration étant en quantité suffisante pour ne pas masquer l'effet de couleur desdites fibres, dans une composition comprenant un milieu physiologiquement acceptable, pour obtenir un maquillage couvrant et présentant un effet de couleur par interférence optique.

L'invention a aussi pour objet un procédé cosmétique de maquillage des matières kératiniques comprenant l'application sur les matières kératiniques d'une première couche, appelée aussi couche de base, d'une première composition cosmétique comprenant dans un milieu cosmétiquement acceptable au moins un agent de coloration, puis l'application sur au moins une partie de ladite première couche, d'une deuxième couche d'une deuxième composition cosmétique comprenant, dans un milieu cosmétiquement acceptable, des fibres interférentielles et un deuxième agent de coloration additionnel, ledit agent de coloration étant en quantité suffisante pour ne pas masquer l'effet de couleur desdites fibres, la première composition ne comprenant pas de particules interférentielles comme présentes dans la deuxième composition.

L'invention a également pour objet un kit de maquillage comprenant :

- une première composition comprenant, dans un milieu cosmétiquement acceptable, un premier agent de coloration, et

- une deuxième composition comprenant, dans un milieu cosmétiquement acceptable, des fibres interférentielles et un deuxième agent de coloration additionnel, ledit agent de coloration étant en quantité suffisante pour ne pas masquer l'effet de couleur desdites fibres,
  la première composition ne comprenant pas de particules interférentielles comme présentes dans la deuxième composition,
  les première et deuxième compositions étant conditionnées dans des récipients distincts.

L'invention a également pour objet un support maquillé, tels que les accessoires de maquillage cités précédemment, comprenant un maquillage susceptible d'être obtenu selon le procédé de maquillage tel que défini précédemment et appliqué sur ledit support.

Les fibres interférentielles conférant un effet visuel original, lorsqu'elles sont formulées dans un support transparent ou translucide, la composition peut être appliquée comme top coat sur un maquillage déjà déposé sur les matières kératiniques pour ainsi modifier l'aspect du maquillage. En outre, l'effet de couleur particulier du maquillage est bien visible lorsque la composition est appliquée sur des peaux sombres comme les peaux éthniques.

Dans la présente demande, on entend par "milieu physiologiquement acceptable", un milieu compatible avec les matières kératiniques d'êtres humains telles que la peau, les ongles, les cheveux, les cils, les sourcils, comme un milieu cosmétique.

Les fibres interférentielles sont des fibres organiques.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 1,2 à 2 500, de préférence de 1,5 à 500, et mieux de 1,6 à 150.

Les fibres utilisables dans la composition de l'invention peuvent être des fibres d'origine synthétique, organique, et plus particulièrement des fibres de polymère synthétique.

En particulier, les fibres interférentielles peuvent être des fibres à structure multicouche de polymère, lesdites couches étant telles qu'elles permettent la création d'un effet de couleur par interférences des rayons lumineux, qui diffractent et diffusent différemment selon les couches. Ainsi de telles fibres peuvent présenter des couleurs variant selon l'angle d'observation, et l'incidence de la lumière, et peuvent conférer des reflets irisés. Des fibres à structure multicouche de polymères sont notamment décrites dans les documents EP-A-921217, EP-A-686858 et US-A-5472798.

La structure multicouche peut comporter au moins deux couches, chaque couche, indépendamment ou non de la (ou les) autre(s) couche(s), étant réalisée en au moins un polymère de synthèse. ,

La fibre à structure multicouche présente de préférence un spectre de réflexion tel que la largeur à mi-hauteur du spectre $\lambda_{L=1/2}$ est dans la gamme $0 < \lambda_{L=1/2} < 200$ nm.

La fibre interférentielle peut être formée de couches individuelles alternées de polymères ayant des indices de réfraction différents ; chaque couche étant dans un plan (P) parallèle à la direction de l'axe principal de la fibre, dans le sens de sa longueur L. Selon l'épaisseur de chacune des différentes couches, on obtient différentes couleurs. En général, la structure est composée d'une alternance de couches de bas indice de réfraction et de haut indice de réfraction. Ainsi, en coupe transversale à la direction de l'axe de la longueur L de la fibre, la fibre a une structure multicouche comprenant des couches alternées d'au moins un premier polymère et un deuxième polymère.

La partie multicouche de la fibre peut comprendre au moins 5 couches individuelles de polymère, notamment de 5 à 120, de préférence au moins 10 couches, notamment de 10 à 70 couches, et mieux de 10 à 50 couches.

Chaque couche des premier et deuxième polymères a respectivement une épaisseur $d_1$, $d_2$ qui peut aller, indépendamment l'une de l'autre, de 0,02 $\mu$m à 0,3 $\mu$m, et de préférence de 0,05$\mu$m à 0,15$\mu$m.

Avantageusement, les polymères présents dans les fibres ont avantageusement un indice de réfraction allant de 1,30 à 1,82 et mieux allant de 1,35 à 1,75. En particulier, les premier et deuxième polymères ont respectivement un indice de réfraction $n_1$ et $n_2$ tels que $n_1/n_2$ va de 1,1 à 1,4.

Avantageusement, $n_1$, $n_2$, $d_1$, $d_2$ satisfont à l'équation :

$$\lambda = 2(n_1 d_1 + n_2 d_2) = 2\, n_1[d_1 + d_2(n_2/n_1)]$$

dans laquelle $\lambda$ est la longueur d'onde, exprimée en $\mu$m, de la couleur de la fibre formée par interférence optique (longueur d'onde du pic du spectre de réflexion) ; $d_1$ et $d_2$ étant exprimés en $\mu$m.

Les fibres utilisables dans la composition selon l'invention sont préférentiellement des fibres de polyester, de polymère acrylique, de polyamide.

Les polymères constituant les fibres particulièrement préférés sont les polyesters tels que le polyéthylène téréphtalate, le polyéthylène naphtalate, le polycarbonate ; les polymères acryliques comme le polyméthacrylate de méthyle ; les polyamides.

Le polyéthylène téréphtalate peut être obtenu par polycondensation d'acide téréphtalique et d'éthylèneglycol. Il peut contenir de 0 à 30% en poids, de préférence de 0 à 15 % en poids, et mieux due 0 à 10% en poids, par rapport au poids total de monomères, d'autres comonomères.

En particulier, le polyéthylène téréphtalate peut comprendre de 0,3 à 10 % en mole, et de préférence de 0,5% à 5% en mole, de monomère diacide carboxylique comportant un groupe acide sulfonique neutralisé par un sel métallique, par rapport au poids total de monomère diacide carboxylique présent dans le polyéthylène téréphtalate.

Le groupe acide sulfonique neutralisé par un sel de métallique est un groupe de formule -$SO_3M$ dans lequel M est un métal, de préférence un métal alcalin ou alcalino-terreux, et plus particulièrement le sodium, le potassium ou le lithium.

Comme exemple de monomère diacide carboxylique comportant un groupe acide sulfonique neutralisé par un sel métallique, on peut utiliser le sel de sodium de l'acide 5-sulfoisophtalique, le sel de potassium de l'acide 5-sulfoisophtalique, le sel de lithium, de l'acide 5-sulfoisophtalique, et leurs diesters méthylique, le sel de sodium de l'acide 5-sulfoisophtalique, le sel de potassium de l'acide 5-sulfoisophtalique, le sel de lithium de l'acide 5-sulfoisophtalique, le 3,5-di($\beta$-hydroxyéthoxycarbonyl)benzènesulfonate de sodium, le 3,5-di($\beta$-hydroxyéthoxy carbonyl)benzènesulfonate de potassium, le 3,5-di($\beta$-hydroxyéthoxycarbonyl) benzènesulfonate de lithium, le diester méthylique de l'acide 4-sulfonate-2,6-naphtalique de sodium, le diester méthylique de l'acide 4-sulfonate-2,6-naphtalique de potassium, le diester méthylique de l'acide 4-sulfonate-2,6-naphtalique de lithium, le 2,6-dicarboxynaphtalène-4-sulfonate de sodium, le 2,6-dicarboxynaphtalène-1-sulfonate de sodium, le diester méthylique de l'acide 3-sulfonate-2,6-naphtalique, le diester méthylique de l'acide 4,8-disulfonate-2,6-naphtalique de sodium, le 2,6-dicarboxynaphtalène-4,8-disulfonate de sodium, le 2,5-bis(hydroxyéthoxy) benzènesulfonate de sodium, le sulfosuccinate de sodium, et leurs mélanges. On utilise de préférence le diester méthylique de l'acide 5-sulfoisophtalique de sodium, le sel de sodium de l'acide 5-sulfoisophtalique et le 3,5-di($\beta$-hydroxyéthoxycarbonyl) benzènesulfonate de sodium.

Le polyéthylène naphtalate peut être obtenu par polycondensation d'acide 2-6-naphtalique ou d'acide 2,7-d'acide naphtalique et d'éthylène glycol. Le polyéthylène naphtalate peut donc être un polyéthylène-2,6-naphtalate ou un polyéthylène-2,7-naphtalate, de préférence un polyéthylène-2,6-naphtalate.

Il peut contenir de 0,3 % à 5 % en mole de monomère diacide carboxylique comportant un groupe acide sulfonique neutralisé par un sel métallique tel que défini précédemment, par rapport au poids total de monomère diacide carboxylique présent dans le poléthylène naphtalate.

D'autres comonomères tels qu'un diacide carboxylique additionnel, différents des diacides carboxyliques mentionnés précédemment, ou un diol additionnel, différent de polyéthylène glycol, peuvent être présents dans le polyéthylène téréphtalate ou le polyéthylène naphtalate.

Le diacide carboxylique additionnel peut être choisi parmi les diacides carboxyliques aromatiques tels que l'acide isophtalique, l'acide biphényl dicarboxylique, l'acide 4,4'-dicarboxylique de diphényléther, l'acide 4,4'-dicarboxylique de diphénylméthane, l'acide 4,4'-dicarboxylique de diphénylsulfone, l'acide 4,4'-dicarboxylique de 1,2-diphénoxyéthane, l'acide 2,5-dicarboxylique de pyridine, l'acide 2,6-dicarboxylique naphtalène, l'acide 2,7-dicarboxylique naphtalène, le diacide carboxylique de diphénylcétone ; les diacides carboxyliques aliphatiques tels que l'acide malonique, l'acide succinique, l'acide adipique, l'acide azélaïque, l'acide sébacique ; les acides dicarboxyliques alicycliques tels que le diacide carboxylique de décaline ; les acides hydroxycarboxyliques tels que l'acide $\beta$-hydroxyéthoxybenzoïque, l'acide para-hydroxybenzoïque et l'acide hydroxypropionique.

Le diol additionnel peut être notamment choisi parmi les diols aliphatiques tels que le propylèneglycol, le butylèneglycol, l'hexylèneglycol, le diéthylèneglycol, le polyéthylèneglycol ; les diols aromatiques tels que l'hydroquinone, le catéchol, le naphtalènediol, la résorcine, le bisphénol A ; les diols alicycliques tels que le cyclohexanediméthanol.

D'autres comonomères peuvent être également choisis parmi les acides carboxyliques polyvalents tels que l'acide trimellitique, l'acide pyromellitique, l'acide tricarballylique ; les alcools polyhydriques tels que la glycérine, le triméthyloléthane, le triméthylolpropane et le pentaérythritol.

Le polyméthylméthacylate peut comprendre des monomères acides tels que l'indice d'acide du polymère soit de préférence supérieur à 3, notamment allant de 3 à 20, et mieux de 4 à 15. De tels monomères acides peuvent être l'acide (méth)acrylique ou l'acide maléique.

Le polyamide peut être choisi parmi le nylon 6, nylon 6-6, nylon 6-12, nylon 11, nylon 12, dont la composition chimique est bien connue de l'homme du métier.

Avantageusement, dans la structure multicouche des fibres plates, le premier polymère peut être choisi parmi les polyesters tels que le polyéthylène téréphtalate, le polyéthylène naphtalate, le polycarbonate, notamment ceux définis précédemment ; le deuxième polymère peut être choisi parmi les polymères acryliques comme le polyméthacrylate de méthyle, et les polyamides, notamment ceux décrits précédemment.

Par ailleurs, les fibres peuvent être traitées ou non en surface, enrobées d'une couche de protection ou non.

Les fibres à structure multicouche peuvent comporter une couche de protection qui peut comprendre un polymère choisi parmi les polymères de couche. De préférence, le polymère de la couche de protection peut avoir un indice de réfraction allant de 1,35 à 1,55.

L'épaisseur de la couche de protection peut être plus grande que l'épaisseur des couches de polymères de la partie multicouche.

L'épaisseur de la couche de protection peut aller de 2 $\mu$m à 10 $\mu$m, et de préférence de 2 $\mu$m à 7 $\mu$m.

Comme polymère de la couche de protection, on peut notamment utiliser le polytétrafluoroéthylène, les copolymères tétrafluoroéthylène/propylène, les copolymères tétrafluoroéthylène/hexafluoropropylène, les copolymères tétrafluoroéthylène/éthylène, les copolymères tétrafluoroéthylène / tétrafluoro propylène, le polyfluorure de vinylidène, les polyacrylates de pentadécafluorooctyl, les polyacrylates de fluoroéthyle, les polyméthacrylates de trifluoroisopropyle, les polyméthacrylates de trifluoroéthyle, les polyacrylates d'éthyle, les polyméthacrylates d'éthyle. On peut également utiliser dés polymères siliconés tels que les polydiméthylsilanes, les les polydiméthylsiloxanes ; des polyuréthanes.

Les fibres interférentielles peuvent être obtenues de façon connue par extrusion du ou des polymères à travers une filière de forme rectangulaire puis découpage du fil obtenu à la longueur voulue.

Les fibres peuvent être unitaires (ou monofilament) ou organisées par exemple tressés (ou multi-filaments). Lorsque les fibres sont des fibres multifilaments, chaque filament peut être de composition chimique différente et présenter une couleur différente : on obtient ainsi des fibres multifilaments présentant des couleurs différentes. En particulier; leurs extrémités sont épointées et/ou polies pour éviter de se blesser.

Avantageusement, les fibres sont insolubles dans l'eau.

La fibre peut être torsadée le long de l'axe de la longueur L de la fibre. Lorsque la fibre n'est pas torsadée, elle présente une couleur dans un certain angle de vue, en dehors de cet angle la fibre est transparente ou de couleur blanche. La fibre plate torsadée quant à elle présente une couleur quel que soit l'angle d'observation.

En particulier, les fibres ont une longueur allant de 1 $\mu$m à 10 mm, de préférence de 0,1 mm à 5 mm et mieux de 0,3 mm à 3,5 mm. Leur section peut être comprise dans un cercle de diamètre allant de 2 nm à 500 $\mu$m, de préférence allant de 100 nm à 100 $\mu$m et mieux de 1$\mu$m à 70 $\mu$m.

Avantageusement, les fibres ont une section transversale (section perpendiculaire à l'axe de la direction de la longueur de la fibre) présentant une plus grande longueur L1 et une plus petite longueur L2 (L2 correspond à l'épaisseur de la fibre) telle que L1/L2 (le rapport L1/L2 est encore appelé facteur d'aplatissement) est supérieur ou égal à 4, de préférence supérieur à 7. Notamment, L1/L2 va de 4 à 15, de préférence de 6 à 12 , et mieux de 7 à 10. Ainsi, la section transversale de la fibre présente une forme plate. Avantageusement, la plus grande longueur L1 et la plus petite longueur L2 définissent respectivement des axes X1, X2 tels que l'axe X1 est sensiblement perpendiculaire à l'axe X2. La plus grande longueur L1 correspond au diamètre D de la fibre tel que mentionné précédemment. Les fibres peuvent notamment présenter une section transversale de forme sensiblement rectangulaire, ovoïdale ou ellipsoïdale. Les fibres peuvent se présenter sous la forme de ruban ou de tagliatelle.

Le poids ou titre des fibres est souvent donné en denier ou décitex et représente le poids en gramme pour 9 km de fil. De préférence, les fibres selon l'invention ont un titre choisi dans la gamme allant de 0,15 à 30 deniers et mieux de 0,18 à 18 deniers.

Comme fibres interférentielles, on peut utiliser les fibres vendues sous les dénominations "Morphotex" , « Teijin Tetron Morphotex » par la société TEIJIN. De telles fibres sont décrites dans la demande EP-A-921217.

Dans un second mode de réalisation de la composition selon l'invention, les particules interférentielles peuvent être des pigments goniochromatiques interférentiels. Ces pigments ont une forme de particules distinctes des fibres interférentielles décrites précédemment.

Par pigment goniochromatique à structure multicouche interférentielle, appelé pigment goniochromatique interférentiel selon l'invention, on entend un pigment à structure au moins bicouche, lesdites couches étant telles qu'elles permettent la création d'un effet de couleur par interférences des rayons lumineux, qui diffractent et diffusent différemment selon les couches. Ainsi de tels pigments peuvent présenter des couleurs variant selon l'angle d'observation et l'incidence de la lumière, et peuvent conférer des reflets irisés.

La structure multicouche peut comporter au moins deux couches, chaque couche, indépendamment ou non de la (ou les) autre(s) couche(s), étant réalisée en au moins un matériau choisi dans le groupe constitué par les matériaux suivants :

$MgF_2$, $CeF_3$, $ZnS$, $ZnSe$, $Si$, $SiO_2$, $Ge$, $Te$, $Fe_2O_3$, $Pt$, $Va$, $Al_2O_3$, $MgO$, $Y_2O_3$, $S_2O_3$, $SiO$, $HfO_2$ $ZrO_2$, $CeO_2$, $Nb_2O_5$, $Ta_2O_5$, $TiO_2$, $Ag$, $Al$, $Au$, $Cu$, $Rb$, $Ti$, $Ta$, $W$, $Zn$, $MoS_2$, cryolithe, alliages, polymères et leurs associations.

De préférence, le pigment goniochromatique à structure multicouche interférentielle selon l'invention est choisi dans le groupe constitué par les pigments goniochromatiques commerciaux suivants : Infinite Colors de SHISEIDO, Sicopearl Fantastico de BASF, Colorstream de MERCK, Colorglitter de 3M, et Chromaflair de FLEX.

Par suite la structure multicouche peut être essentiellement minérale ou organique. Selon l'épaisseur de chacune des différentes couches, on obtient différentes couleurs.

Les pigments à structure multicouche interférentielle selon l'invention sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479 (BASF), DE-A-196 14 637, et leurs associations. Ils se présentent sous forme de paillettes, de couleur métallisée.

Par exemple, la structure multicouche interférentielle est choisie dans le groupe constitué par les structures : $Fe_2O_3/SiO_2/Fe_2O_3/SiO_2/Fe_2O_3$; § $MoS_2/SiO_2/micaoxyde/SiO_2/M_OS_2$; $Fe_2O_3/SiO_2/mica\text{-}oxyde/SiO_2/Fe_2O_3$.

En général, la structure est composée d'une alternance de couches de bas indice optique et haut indice optique.

Les particules interférentielles peuvent être présentes dans la composition selon l'invention, ou dans la composition de surface, en une teneur allant de 0,01 % à 50 % en poids, par rapport au poids total de la composition, de préférence de 0,1 % à 30 % en poids, et mieux de 0,3 % à 20% en poids.

L'agent de coloration présent dans la composition selon l'invention, ou dans la composition de base et/ou de surface, est différent des particules interférentielles décrites précédemment. L'agent de coloration additionnel peut être choisi parmi les pigments, les nacres, les colorants, et leurs mélanges.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être présents dans la composition, notamment dans la composition de base et/ou de surface, à raison de 0 à 15 % (notamment 0,01 % à 15 %) par rapport au poids de la composition, de préférence de 0,01 % à 10 % en poids, et mieux de 0,02 % à 5 % en poids.

Les pigments peuvent être blancs ou colorés, minéraux et/où organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les nacres peuvent être présentes dans la composition, notamment dans la composition de base et/ou de surface, à raison de 0 à 25 % (notamment 0,01% à 25 %) en poids, par rapport au poids total de la composition, de préférence de 0,01% à 15 % en poids, et mieux de 0,02 % à 5 % en poids.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

L'agent de coloration peut également être une matière colorante choisie parmi les colorants hydrosolubles ou liposolubles ou bien encore les polymère colorants. La matière colorante peut être présente dans la composition, notamment la composition de base et/ou de surface, en une teneur en matière active de colorant allant de 0 à 6 % (notamment 0,01 % à 6 %) en poids, par rapport au poids total de la composition, de préférence allant de 0,01 % à 3 % en poids.

Les colorants liposolubles sont par exemple l'huile de soja, le brun Soudan, le DC Yellow 11, le DC orange 5, le jaune quinoléine, le Rouge Soudan III (nom CTFA D&C red 17), la lutéine, le vert de quinizarine (nom CTFA DC green 6), le pourpre d'Alizurol SS (nom CTFA DC violet N°2), les dérivés caroténoïdes comme le lycopène, le béta-carotène, la bixine, la capsantéine, et/ou leurs mélanges.

Parmi les colorants hydrosolubles, on peut citer les extraits de plantes tinctoriales, comme par exemple l'Aleurites Moluccana Willd, l'Alkanna Tinctoria Tausch, l'Areca Catechu L., l'Arrabidaea Chica E. et B., la Bixa Orellana L (rocou), la Butea Monosperma Lam, la Caesalpina Echinata Lam, la Caesalpina Sappan L., la Calophyllum Inophyllum L., la Carthamus Tinctorius L., la Cassia Alata L., la Chrozophora Tinctoria L., La Crocus Sativus L., la Curcuma Longa L., la Diospyros Gilletii de Wild, l'Eclipta Prostrata L., la Gardenia Erubescens Stapf. et Hutch., la Gardenia Terniflora Schum. et Thonn., la Genipa Americana L., la Genipa Brasiliensis L., la Guibourtia Demeusei (Harms) J. Leon, l'Haematoxylon Campechianum L., l'Helianthus annuus, l'Humiria Balsamifera (Aubl.) St-Hil., l'Isatis Tinctoria L., le Mercurialis perenis, le Monascus purpureus, le Monascus ruber, le Monascus pilosus, la Morus Nigra L., la Picramnia Spruceana, la Pterocarpus Erinaceus Poir., la Pterocarpus Soyauxii Taub., la Rocella Tinctoria L., la Rothmannia Whitfieldii (Lindl.) Dand., la Schlegelia Violacea (Aubl.) Griseb., la Simira Tinctoria Aublet, la Stereospermum Kunthianum Cham., la Symphonie Globulifera L., la Terminalia Catappa L., la Sorgho, l'Aronia melanocarpa, les naphtoquinone dont la lawsone, issue de la Lawsonia Inermis L. encore appelée henné ou de l'Impatiens Balsamina, les extraits de bois rouge tels que décrits dans le document WO98/44902 le jus de betterave, le sel disodique de suschine, les anthocyanes comme les extraits de fruits rouges, la dihydroxyacétone, les dérivés mono ou polycarbonylés tels que l'isatine, l'alloxane, la ninhydrine, le glycéraldéhyde, l'aldéhyde mésotartrique, les dérivés de pyrazoline-4,5-dione, et leurs mélanges, ces agents de coloration de la peau pouvant être associés ou non à des colorants directs ou des dérivés indoliques, et/ou leurs mélanges.

Ces extraits de plantes tinctoriales peuvent se trouver sous forme de lyophilisat, de pâte, ou encore de solution : généralement, on broie les feuilles de la plante tinctoriale pour obtenir une poudre. On met cette poudre en solution dans une phase aqueuse durant quelques heures. Le mélange est ensuite centrifugé puis filtré. Le filtrat obtenu est congelé puis lyophilisé.

L'agent de coloration peut également être un polymère colorant, c'est-à-dire un polymère comportant au moins un groupement colorant organique. Le polymère colorant contient en général moins de 10% en poids, par rapport au poids total du polymère, de matière colorante.

Le polymère colorant peut être de toute nature chimique, notamment polyester, polyamide, polyuréthanne, polyacrylique, poly(méth)acrylique, polycarbonate, polymères d'origine naturelle comme les polymères cellulosiques ou de chitosane, ou leurs mélanges, et de préférence des polymères polyester ou polyuréthanne.

Le polymère colorant peut comprendre un groupement colorant peut être greffé, notamment par laiison covalente, sur la chaîne du polymère, comme décrit dans les documents WO-A-96/29046, WO-A-92/01022, WO-A-90/07558, BE-A-609054.

En particulier, le polymère colorant peut être un copolymère à base d'au moins deux monomères distincts dont l'un au moins est un monomère colorant organique.

Les monomères du polymère colorant peut être choisi parmi les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines; les phtaloylphénoxazines, les phtaloylacridone, les anthrapyrimidines, les anthrapyrazoles, les phtalocyanines, les quinophtalones, les indophénols, perinones, les nitroarylamines, benzodifurane, les 2H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine, oxazine. Des monomères colorants sont notamment décrits dans les documents US-A-4,267,306 ; US-A-4,359,570 ; US-A-4,403,092 ; US-A-4,617,373 ; US-A-4,080,355 ; US- A-4,740,581 ; US-A-4,116,923 ; US-A-4,745,173 ; US-A-4,804,719, US-A-5, 194,463 ; US-A-5,804,719 ; WO-A-92/07913.

Des colorants polymériques sont notamment décrits dans les documents US-A-4,804,719 ; US-A-5,032,670 ; US-A-4,999,418 ; US-A-5,106,942 ; US-A-5,030,708 ; US-A-5,102,980 ; US-A-5,043,376, US-A-5,194,463; WO-A-92/07913 ; WO-A-97/24102.

On utilise de préférence des polymères colorants sulfopolyesters tels que ceux décrits dans le document WO-A-97/24102.

Les polymères colorants peuvent être présents dans la composition selon l'invention, notamment dans la composition de base et/ou de surface, en une teneur allant de 0 % à 50 % en poids (0,01% à 50%), par rapport au poids total de la composition, de préférence allant de 0,5% à 25% en poids, et mieux allant de 0,2% à 20% en poids.

Avantageusement, les particules interférentielles et l'agent de coloration additionnel peuvent être présents dans la composition selon l'invention, ou dans la composition de surface, selon un rapport pondéral particules interférentielles / matière active de l'agent de coloration additionnel supérieur ou égal à 2 (notamment allant de 2 à 500), et mieux supérieur ou égal à 5 (notamment allant de 5 à 500).

En plus de l'agent de coloration additionnel, la composition selon l'invention peut contenir en outre des charges. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acryliques (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes à raison de 0 à 90 % en poids, par rapport au poids total de la composition, notamment de la composition de base et/ou de surface, de préférence 0,01 à 50 % en poids, et mieux de 0,02 % à 30 % en poids.

La composition de l'invention, ou la composition de base et/ou de surface, peut comprendre une phase particulaire comprenant les pigments et/ou les nacres et/ou les charges tels que définis précédemment, pouvant être présente à raison de 0 à 98 % (notamment 0,01 % à 98 %) du poids total de la composition, de préférence de 0,01 % à 30 % et mieux de 0,02 % à 20 %.

La composition selon l'invention, ou l'une des compositions de base et/ou de surface, peut comprendre un milieu cosmétique hydrophile ou un milieu lipophile.

La composition, ou l'une des compositions de base et/ou de surface, peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La phase hydrophile peut, en outre, contenir des éthers en $C_2$ et des aldéhydes en $C_2$-$C_4$ hydrophiles. L'eau ou le mélange d'eau et de solvants organiques hydrophiles peut être présent dans la composition selon l'invention, ou l'une des compositions de base et/ou de surface, en une teneur allant de 0 % à 90 % (notamment 0,1 % à 90%) en poids, par rapport au poids total de la composition, et de préférence de 0 % à 60 % en poids (notament 0,1 % à 60 % en poids).

La composition, ou l'une des compositions de base et/ou de surface, peut également comprendre une phase grasse, notamment constituée de corps gras liquides à température ambiante (25°C en général) et/ou de corps gras solides à température ambiante tels que les cires, les corps gras pâteux, les gommes et leurs mélanges. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le pérhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.

Ces huiles peuvent être présentes en une teneur allant de 0,01 à 90%, et mieux de 0,1 à 85 % en poids, par rapport au poids total de la composition,

La composition, ou l'une des compositions de base et/ou de surface, selon l'invention peut également comprendre un ou plusieurs solvants organiques, cosmétiquement acceptables (tolérance, toxicologie et toucher acceptables). Ces solvants peuvent être présents en une teneur allant de 0 à 90 % et mieux de 0 à 60% en poids, par rapport au poids total de la composition et mieux de 0,1 à 30 %. Comme solvants utilisables dans la composition de l'invention, on peut citer les esters de l'acide acétique comme l'acétate de méthyle, d'éthyle, de butyle, d'amyle, de méthoxy-2-éthyle, l'acétate d'isopropyle; les cétones comme la méthyléthylcétone, la méthylisobutylcétone ; les hydrocarbures comme le toluène, le xylène, l'hexane, l'heptane ; les aldéhydes ayant de 5 à 10 atomes de carbone ; les éthers ayant au moins 3 atomes dé carbones ; et leurs mélanges.

La composition de l'invention, ou l'une des compositions de base et/ou de surface, peut en outre comprendre, avantageusement un corps gras solide ou pâteux à température ambiante, comme les gommes ou les cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45°C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxydiméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.

La composition selon l'invention, ou l'une des compositions de base et/ou de surface, peut en outre comprendre un polymère filmogène. Dans la présente demande, on entend par "polymère filmogène", un polymère apte à former à lui seul ou en présence d'un agent auxiliaire de filmification, un film continu et adhérent sur un support, notamment sur les matières kératiniques.

Le polymère filmogène peut être choisi parmi les polymères vinyliques, les polycondensats ou les polymères d'origine naturelle. Comme polymère filmogène, on peut citer en particulier les polymères acryliques, les polyuréthanes, les polyesters, les polyamides, les polyurées, les polymères cellulosiques. Le polymère filmogène peut être dissous ou dispersé sous forme de particules solides dans le milieu physiologiquement acceptable de la composition.

Le polymère filmogène peut être présent dans la composition selon l'invention, ou l'une des compositions de base et/ou de surface, en une teneur en matières sèches de polymère allant de 0,01% à 60% en poids par rapport au poids total de la composition, de préférence de 0,5 % à 40 % en poids, et mieux de 1 % à 30 % en poids.

Le polymère filmogène peut être associé à des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention, ou l'une des compositions de base et/ou de surface, peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E. ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de $200s^{-1}$, après 10 minutes de mesure en géométrie cône/plan). La composition peut être à phase continue organique, notamment anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention, ou l'une des compositions de base et/ou de surface, peut également contenir des ingrédients couramment utilisés en cosmétique, tels que les vitamines, les épaississants (notamment les argiles éventuellement modifiées), les oligo-éléments, les adoucissants, les séquestrants, les parfums, les agents alcalinisants ou acidifiants, les conservateurs, les filtres UV, ou leurs mélanges.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition de l'invention , notamment les compositions de base et de surface, peut être obtenue selon les procédés de préparation classiquement utilisés en cosmétique ou en dermatologie.

**Exemple 1 :**

On a préparé une poudre libre pour le visage comprenant :

- Poudre de nylon-12          30g
- Fibres interférentielles de polyéthylène téréphtalate et de nylon de longueur 0,3 mm vendues sous la dénomination "Morphotex" par la société TEIJIN          10 g

- Oxydes de fer          3,5 g
- liant siliconé          3 g
- Talc          qsp          100 g

La poudre appliquée sur le visage confère un maquillage lumineux.

### Exemple 2 :

On a préparé un fond de teint ayant la composition suivante :

- mélange cétyl diméthicone copolyol /polyglycéryl-4-isostéarate / hexyl laurate vendu sous la dénomination commerciale "Abil WE 09" par la Société Goldschmidt0,          5g
- Fibres interférentielles de polyéthylène téréphtalate et de nylon de longueur 0,3 mm vendues sous la dénomination "Morphotex" par la société TEIJIN          10 g
- Oxydes de fer          0,5 g
- Silicone volatile (DC245 Fluid de la société Dow Corning)          15g
- Eau          qsp          100 g

Le fond de teint appliqué sur le visage confère un maquillage illuminateur du teint.

### Exemple 3 :

On a préparé un mascara ayant la composition suivante :

- Carboxyméthyl cellulose          15 g
- Laponite          0,2 g
- Fibres interférentielles de polyéthylène téréphtalate et de nylon de longueur 0,3 mm vendues sous la dénomination "Morphotex" par la société TEIJIN          10 g
- Sel disodique de suschine          0,05 g
- eau          qsp          100 g

Les cils maquillés avec ce mascara présentent un effet de couleur original.

### Exemple 4 :

On a préparé un vernis à ongles ayant la composition suivante :

- nitrocellulose          17,1 g
- N-éthyl o,p-toluènesulfonamide          5,4 g
- acétyl citrate de tributyle          5,4 g
- Fibres interférentielles de polyéthylène téréphtalate et de nylon de longueur 0,3 mm vendues sous la dénomination "Morphotex" par,la société TEIJIN          10 g
- DC Red 34          0,025 g
- hectorite          1,0 g
- alcool isopropylique          7,2g
- acétate d'éthyle, acétate de butyle          qsp 100 g

Ce vernis à ongles peut être appliqué directement sur les ongles ou bien sur une couche de base obtenue après l'application d'une base de vernis à ongles de composition suivante :

- nitrocellulose          19g
- N-éthyl o,p-toluènesulfonamide          6 g
- acétyl citrate de tributyle          6 g
- pigments bleu nuit          1g
- hectorite          1,2 g
- alcool isopropylique          8 g
- acétate d'éthyle, acétate de butyle          qsp 100 g

On obtient un maquillage des ongles présentant un effet de couleur sur fond bleu nuit.

**Revendications**

1. Composition comprenant, dans un milieu physiologiquement acceptable, des fibres interférentielles de polymère synthétique présentant un effet de couleur, ayant une section transversale de forme ovoïdale ou ellipsoïdale et une longueur L et un diamètre D tel que L/D est choisi dans la gamme allant de 1,2 à 2 500, et un agent de coloration additionnel, ledit agent de coloration étant présent en quantité telle que le rapport pondéral fibres interférentielles / matière active de l'agent de coloration additionnel soit de 2 à 500.

2. Composition selon la revendication précédente, **caractérisée par le fait que** les fibres interférentielles sont choisies parmi les fibres de polyester, de polymère acrylique, de polyamide.

3. Composition selon l'une quelconque des revendications précédentes, **caractérisée par le fait que** les fibres interférentielles comprennent un polymère choisi dans le groupe formé par le polyéthylène téréphtalate, le polyéthylène naphtalate, le polycarbonate, le polyméthacrylate de méthyle, le nylon 6, nylon 6-6, nylon 6-12, nylon 11, nylon 12.

4. Composition selon l'une quelconque des revendications précédentes, **caractérisée par le fait que** les fibres Interférentielles sont des fibres à structure multicouche de polymères comprenant des couches alternées d'au moins un premier polymère et un deuxième polymère.

5. Composition selon la revendication précédente, **caractérisée par le fait que** chaque couche de polymère étant dans un plan (P) parallèle à la direction de l'axe principal de la fibre, dans le sens de sa longueur L.

6. Composition selon l'une quelconque des revendications 4 à 5, **caractérisée par le fait que** la partie multicouche de la fibre comprend au moins 5 couches individuelles de polymère.

7. Composition selon l'une quelconque des revendications 4 à 6, **caractérisée par le fait que** la partie multicouche de la fibre comprend de 5 à 120 couches individuelles de polymère.

8. Composition selon l'une quelconque des revendications 4 à 7, **caractérisée par le fait que** la partie multicouche de la fibre comprend de 10 à 50 couches individuelles de polymère,

9. Composition selon l'une quelconque des revendications 4 à 8, **caractérisée par le fait que** chaque couche des premier et deuxième polymères a respectivement une épaisseur $d_1$, $d_2$ allant, indépendamment l'une de l'autre, de 0,02 $\mu$m à 0,3 $\mu$m,

10. Composition selon l'une quelconque des revendications 4 à 9, **caractérisée par le fait que** les polymères présents dans les fibres ont un indice de réfraction allant de 1,30 à 1,82.

11. Composition selon l'une quelconque des revendications 4 à 10, **caractérisée par le fait que** les premier et deuxième polymères ont respectivement un indice de réfraction $n_1$ et $n_2$ tels que $n_1/n_2$ va de 1,1 à 1,4,

12. Composition selon l'une quelconque des revendications 4 à 11, **caractérisée par le fait que** la fibre à structure multicouche présente un spectre de réflexion tel que la largeur à mi-hauteur du spectre $\lambda_{L=1/2}$ est dans la gamme $0 < \lambda_{L=1/2} < 200$ nm.

13. Composition selon l'une quelconque des revendications 4 à 11, **caractérisée par le fait que** le premier polymère est un polyamide et le deuxième polymère est un polyester.

14. Composition selon l'une quelconque des revendications précédentes, **caractérisée par le fait que** les fibres sont traitées en surface ou enrobées d'une couche de protection.

15. Composition selon la revendication précédente, **caractérisée par le fait que** la couche de protection comprend un polymère choisi dans le groupe formé par les polyuréthanes, les polyacrylates d'éthyle, les polyméthacrylates d'éthyle.

**16.** Composition selon la revendication précédente, **caractérisée par le fait que** le polymère de la couche de protection a un indice de réfraction allant de 1,35 à 1,55.

**17.** Composition selon l'une quelconque des revendications précédentes, **caractérisée par le fait que** les fibres ont une longueur L et un diamètre D tel que L/D est choisi dans la gamme allant de 1,6 à 150.

**18.** Composition selon la revendication précédente, **caractérisée par le fait que** les fibres ont une section transversale comprise dans un cercle de diamètre allant de 2 nm à 500 $\mu$m.

**19.** Composition selon l'une quelconque des revendications précédentes, **caractérisée par le fait que** les fibres ont une longueur L allant de 1 $\mu$m à 10 mm.

**20.** Composition selon l'une quelconque des revendications précédentes, **caractérisée par le fait que** les fibres sont plates.

**21.** Composition selon l'une quelconque des revendications précédentes, **caractérisée par le fait que** les fibres ont une section transversale présentant une plus grande longueur L1 et une plus petite longueur L2 telles que L1/L2 est supérieur à 4.

**22.** Composition selon la revendication précédente, **caractérisée par le fait que** L1/L2 va de 4 à 15.

**23.** Composition selon l'une quelconque des revendications 20 à 22, **caractérisée par le fait que** les fibres plates se présentent sous forme de ruban ou de tagliatelle.

**24.** Composition selon l'une quelconque des revendications précédentes, **caractérisée par le fait que** les fibres sont des fibres monofilament ou multifilaments.

**25.** Composition selon l'une quelconque des revendications précédentes, **caractérisée par le fait que** les fibres sont torsadées le long de l'axe de la longueur L des fibres.

**26.** Composition selon l'une quelconque des revendications précédentes, **caractérisée par le fait que** les fibres ont un titre choisi dans la gamme allant de 0,15 à 30 deniers.

**27.** Composition selon l'une quelconque des revendications précédentes, **caractérisée par le fait que** les fibres interférentielles sont présentes en une teneur allant de 0,01 % à 50 % en poids, par rapport au poids total de la composition.

**28.** Composition selon l'une quelconque des revendications précédentes, **caractérisée par le fait que** les fibres interférentielles sont présentes en une teneur allant de 0,3 % à 20 % en poids, par rapport au poids total de la composition.

**29.** Composition selon l'une quelconque des revendications précédentes, **caractérisée par le fait que** l'agent de coloration additionnel est choisi dans le groupe formé par les pigments, les nacres, les colorants hydrosolubles ou liposolubles, les polymères colorants.

**30.** Composition selon l'une quelconque des revendications précédentes, **caractérisée par le fait que** l'agent de coloration additionnel est un pigment choisi parmi le dioxyde de titane, les oxydes de zirconium, les oxydes de cérium, les oxydes de zinc, les oxydes de fer, les oxydes de chrome, le violet de manganèse, le bleu outremer, l'hydrate de chrome, le bleu ferrique, la poudre d'aluminium, la poudre de cuivre, le noir de carbone, les pigments de type D & C, et les laques à base de carmin de cochenille, de baryum, strontium, calcium, aluminium,

**31.** Composition selon l'une des revendications 29 ou 30, **caractérisée par le fait que** les pigments sont présents en une teneur allant de 0,01 % à 15 % en poids, par rapport au poids de la composition.

**32.** Composition selon l'une des revendications 29 à 31, **caractérisée par le fait que** les pigments sont présents en une teneur allant de 0,02 % à 5 % en poids, par rapport au poids de la composition.

**33.** Composition selon la revendication 29, **caractérisée par le fait que** l'agent de coloration est une nacre choisie parmi le mica recouvert de titane ou d'oxychlorure de bismuth, le mica titane recouvert avec des oxydes de fer, le mica titane recouvert avec du bleu ferrique ou de l'oxyde de chrome, le mica titane recouvert avec un pigment organique, les pigments nacrés à base d'oxychlorure de bismuth.

34. Composition selon l'une des revendications 29 ou 30, **caractérisée par le fait que** les nacres sont présentes en une teneur allant de 0,01 % à 25 % en poids, par rapport au poids de la composition.

35. Composition selon l'une des revendications 29 ou 33, 34, **caractérisée par le fait que** les nacres sont présentes en une teneur allant de 0,02 % à 5 % en poids, par rapport au poids de la composition.

36. Composition selon l'une quelconque des revendications précédentes, **caractérisée par le fait que** l'agent de coloration est choisi parmi les colorants hydrosolubles ou liposolubles et les polymères colorants, et est présent en une teneur en matière active de colorant allant de 0,01 % à 6 % en poids, par rapport au poids total de la composition.

37. Composition selon l'un quelconque des revendications précédentes, **caractérisée par le fait que** les fibres interférentielles et l'agent de coloration additionnel sont présents selon un rapport pondéral fibres interférentielles / matière active de l'agent de coloration additionnel allant de 5 à 500.

38. Composition selon l'une quelconque des revendications précédentes, **caractérisée par le fait qu'**elle comprend un milieu cosmétique hydrophile ou lipophile.

39. Composition selon l'une quelconque des revendications précédentes, **caractérisée par le fait qu'**elle comprend de l'eau ou un mélange d'eau et de solvant organique hydrophile.

40. Composition selon l'une quelconque des revendications précédentes, **caractérisée par le fait qu'**elle comprend une phase grasse.

41. Composition selon la revendication précédente, **caractérisée par le fait que** la composition contient un ingrédient choisi parmi les huiles, les cires, les corps gras pâteux, les gommes, et leurs mélanges.

42. Composition selon l'une quelconque des revendications précédentes, **caractérisée par le fait qu'**elle contient un solvant organique.

43. Composition selon l'une quelconque des revendications précédentes, **caractérisée par le fait qu'**elle comprend un polymère filmogène,

44. Composition selon la revendication précédente, **caractérisée par le fait que** le polymère filmogène est choisi dans le groupe formé par les polymères vinyliques, les polyuréthanes, les polyesters, les polyamides, les polyurées, les polymères cellulosiques.

45. Composition selon l'une des revendications 43 ou 44, **caractérisée par le fait que** le polymère filmogène est présent en une teneur en matières sèches de polymère allant de 0,1 % à 60 % en poids par rapport au poids total de la composition,

46. Composition selon l'une quelconque des revendications précédentes, **caractérisée par le fait qu'**elle comprend un ingrédient cosmétique choisi dans le groupe formé par les charges, les vitamines, les épaississants, les oligo-éléments, les adoucissants, les séquestrants, les parfums, les agents alcallnisants ou acidifiants, les conservateurs, ou leurs mélanges.

47. Composition selon l'une des revendications précédentes, **caractérisée par le fait qu'**elle se présente sous la forme d'un vernis à ongles, de mascara, d'eye-liner, de composition capillaire, de produit pour les lèvres, de fond de teint, de produit anti-cernes, de fard à Joues ou à paupières, de produit pour les sourcils, de produit de maquillage du corps.

48. Composition selon l'une quelconque des revendications précédentes, **caractérisée par le fait qu'**elle se présente sous la forme de vernis à ongles.

49. Procédé cosmétique de maquillage des matières kératiniques, **caractérisé par le fait que** l'on applique sur les matières kératiniques une composition selon l'une quelconque des revendications précédentes.

50. Procédé cosmétique de maquillage des matières kératiniques comprenant l'application sur les matières kératiniques d'une première couche, appelée aussi couche de base, d'une première composition cosmétique comprenant dans un milieu cosmétiquement acceptable au moins un agent de coloration, puis l'application sur au moins une partie de ladite première couche, d'une deuxième couche d'une deuxième composition cosmétique conforme à la composition telle que définie selon l'une quelconque des revendications 1 à 48, la première composition ne comprenant pas de fibres interférentielles comme présentes dans la deuxième composition.

**51.** Procédé selon la revendication précédente, **caractérisé par le fait que** la première composition comprend un agent de coloration choisi parmi les pigments, les nacres, les colorants hydrosolubles ou liposolubles.

**52.** Procédé selon la revendication 50 ou 51, **caractérisé par le fait que** la première composition comprend un polymère filmogène.

**53.** Procédé selon l'une quelconque des revendications 50 à 52, **caractérisé par le fait que** la première composition comprend un ingrédient cosmétique choisi dans le groupe formé par les charges, les vitamines, les épaississants, les oligo-éléments, les adoucissants, les séquestrants, les parfums, les agents alcalinisants ou acidifiants, les conservateurs, ou leurs mélanges.

**54.** Procédé selon l'une quelconque des revendications 50 à 53, **caractérisé par le fait que** la première composition se présente sous la forme d'un vernis à ongles, de mascara, d'eye-liner, de composition capillaire, de produit pour les lèvres, de fond de teint, de produit anti-cernes, de fard à joues ou à paupières, de produit pour les sourcils, de produit de maquillage du corps.

**55.** Procédé selon l'une quelconque des revendications 50 à 54, **caractérisé par le fait que** la première composition se présente sous la forme de vernis à ongles.

**56.** Kit de maquillage comprenant ;

- une première composition cosmétique comprenant, dans un milieu cosmétiquement acceptable, un premier agent de coloration, et
- une deuxième composition cosmétique conforme à la composition telle que définie selon l'une quelconque des revendications 1 à 48,
la première composition ne comprenant pas de particules interférentielles comme présentes dans la deuxième composition,
les première et deuxième compositions étant conditionnées dans des récipients distincts.

**57.** Kit de maquillage selon la revendication précédente, **caractérisé par le fait que** la première composition comprend un agent de coloration choisi parmi les pigments, les nacres, les colorants hydrosolubles ou liposolubles,

**58.** Kit de maquillage selon la revendications 56 ou 57, **caractérisé par le fait que** la première composition comprend un polymère filmogène.

**59.** Kit de maquillage selon l'une quelconque des revendications 56 à 58, **caractérisé par le fait que** la première composition comprend un Ingrédient cosmétique choisi dans le groupe formé par les charges, les vitamines, les épaississants, les oligo-éléments, les adoucissants, les séquestrants, les parfums, les agents alcalinisants ou acidifiants, les conservateurs, ou leurs mélanges.

**60.** Kit de maquillage selon l'une quelconque des revendications 56 à 59, **caractérisé par le fait que** la première composition se présente sous la forme d'un vernis à ongles, de mascara, d'eye-liner, de composition capillaire, de produit pour les lèvres, de fond de teint, de produit anti-cernes, de fard à joues ou à paupières, de produit pour les sourcils, de produit de maquillage du corps.

**61.** Kit de maquillage selon l'une quelconque des revendications 56 à 60, **caractérisé par le fait que** la première composition se présente sous la forme de vernis à ongles.

**62.** Support maquillé comprenant un maquillage susceptible d'être obtenu selon le procédé de maquillage conforme à l'une quelconque des revendications 50 à 55 et appliqué sur ledit support, ledit support étant choisi parmi les faux ongles, les faux cils, les postiches, les perruques, les pastilles ou les patchs adhérents sur la peau ou les lèvres.

**Claims**

1. Composition comprising, in a physiologically acceptable medium, interference fibres of synthetic polymer exhibiting a colour effect, having a transverse cross section of ovoidal or ellipsoidal shape and a length L and diameter D such that L/D is chosen within the range extending from 1.2 to 2500, and an additional colouring agent, the said colouring agent being present in an amount such that the interference fibres/active material of the additional colouring agent ratio by weight is from 2 to 500.

2. Composition according to the preceding claim, **characterized in that** the interference fibres are chosen from polyester, acrylic polymer or polyamide fibres.

3. Composition according to either one of the preceding claims, **characterized in that** the interference fibres comprise a polymer chosen from the group formed by polyethylene terephthalate, polyethylene naphthalate, polycarbonate, polymethyl methacrylate, nylon 6, nylon 6,6, nylon 6,12, nylon 11 and nylon 12.

4. Composition according to any one of the preceding claims, **characterized in that** the interference fibres are fibres with a multilayer structure formed of polymers comprising alternating layers of at least one first polymer and one second polymer.

5. Composition according to the preceding claim, **characterized in that** each polymer layer is in a plane (P) parallel to the direction of the main axis of the fibre, in the direction of its length L.

6. Composition according to either one of Claims 4 and 5, **characterized in that** the multilayer part of the fibre comprises at least 5 individual layers of polymer.

7. Composition according to any one of Claims 4 to 6, **characterized in that** the multilayer part of the fibre comprises from 5 to 120 individual layers of polymer.

8. Composition according to any one of Claims 4 to 7, **characterized in that** the multilayer part of the fibre comprises from 10 to 50 individual layers of polymer.

9. Composition according to any one of Claims 4 to 8, **characterized in that** each layer of the first and second polymers respectively has a thickness $d_1$, $d_2$ ranging, independently of one another, from 0.02 $\mu$m to 0.3 $\mu$m.

10. Composition according to any one of Claims 4 to 9, **characterized in that** the polymers present in the fibres have a refractive index ranging from 1.30 to 1.82.

11. Composition according to any one of Claims 4 to 10, **characterized in that** the first and second polymers respectively have a refractive index $n_1$ and $n_2$ such that $n_1/n_2$ ranges from 1.1 to 1.4.

12. Composition according to any one of Claims 4 to 11, **characterized in that** the fibre with a multilayer structure exhibits a reflectance spectrum such that the width at mid-height of the spectrum $\lambda_{w=1/2}$ is within the range $0 < \lambda_{w=1/2} < 200$ nm.

13. Composition according to any one of Claims 4 to 11, **characterized in that** the first polymer is a polyamide and the second polymer is a polyester.

14. Composition according to any one of the preceding claims, **characterized in that** the fibres are surface-treated or coated with a protective layer.

15. Composition according to the preceding claim, **characterized in that** the protective layer comprises a polymer chosen from the group formed by polyurethanes, polyethyl acrylates and polyethyl methacrylates.

16. Composition according to the preceding claim, **characterized in that** the polymer of the protective layer has a refractive index extending from 1.35 to 1.55.

17. Composition according to any one of the preceding claims, **characterized in that** the fibres have a length L and a diameter D such that L/D is chosen within the range extending from 1.6 to 150.

18. Composition according to the preceding claim, **characterized in that** the fibres have a transverse cross section included within a circle with a diameter ranging from 2 nm to 500 μm.

19. Composition according to any one of the preceding claims, **characterized in that** the fibres have a length L ranging from 1 μm to 10 mm.

20. Composition according to any one of the preceding claims, **characterized in that** the fibres are flat.

21. Composition according to any one of the preceding claims, **characterized in that** the fibres have a transverse cross section exhibiting a greatest length L1 and a smallest length L2 such that L1/L2 is greater than 4.

22. Composition according to the preceding claim, **characterized in that** L1/L2 ranges from 4 to 15.

23. Composition according to any one of Claims 20 to 22, **characterized in that** the flat fibres are provided in the tape or tagliatelle form.

24. Composition according to any one of the preceding claims, **characterized in that** the fibres are monofilament or multifilament fibres.

25. Composition according to any one of the preceding claims, **characterized in that** the fibres are twisted along the axis of the length L of the fibres.

26. Composition according to any one of the preceding claims, **characterized in that** the fibres have a count chosen within the range extending from 0.15 to 30 denier.

27. Composition according to any one of the preceding claims, **characterized in that** the interference fibres are present in a content ranging from 0.01% to 50% by weight, with respect to the total weight of the composition.

28. Composition according to any one of the preceding claims, **characterized in that** the interference fibres are present in a content ranging from 0.3% to 20% by weight, with respect to the total weight of the composition.

29. Composition according to any one of the preceding claims, **characterized in that** the additional colouring agent is chosen from the group formed by pigments, pearlescent agents, water-soluble or fat-soluble dyes, and polymer dyes.

30. Composition according to any one of the preceding claims, **characterized in that** the additional colouring agent is a pigment chosen from titanium dioxide, zirconium oxides, cerium oxides, zinc oxides, iron oxides, chromium oxides, manganese violet, ultramarine blue, chromium hydrate, ferric blue, aluminium powder, copper powder, carbon black, pigments of D & C type and lakes, based on cochineal carmine, of barium, strontium, calcium or aluminium.

31. Composition according to either of Claims 29 and 30, **characterized in that** the pigments are present in a content ranging from 0.01% to 15% by weight, with respect to the weight of the composition.

32. Composition according to one of Claims 29 to 31, **characterized in that** the pigments are present in a content ranging from 0.02% to 5% by weight, with respect to the weight of the composition.

33. Composition according to Claim 29, **characterized in that** the colouring agent is a pearlescent agent chosen from mica covered with titanium oxide or with bismuth oxychloride, titanium oxide-coated mica covered with iron oxides, titanium oxide-coated mica covered with ferric blue or chromium oxide, or titanium oxide-coated mica covered with an organic pigment, or pearlescent pigments based on bismuth oxychloride.

34. Composition according to either of Claims 29 and 33, **characterized in that** the pearlescent agents are present in a content ranging from 0.01% to 25% by weight, with respect to the weight of the composition.

35. Composition according to one of Claims 29, 33 and 34, **characterized in that** the pearlescent agents are present in a content ranging from 0.02% to 5% by weight, with respect to the weight of the composition.

36. Composition according to any one of the preceding claims, **characterized in that** the colouring agent is chosen from water-soluble or fat-soluble dyes and from polymer dyes and is present in a content, as dye active material, ranging from 0.01% to 6% by weight, with respect to the total weight of the composition.

37. Composition according to any one of the preceding claims, **characterized in that** the interference fibres and the additional colouring agent are present according to an interference fibres/active material of the additional colouring agent ratio by weight ranging from 5 to 500.

38. Composition according to any one of the preceding claims, **characterized in that** it comprises a hydrophilic or lipophilic cosmetic medium.

39. Composition according to any one of the preceding claims, **characterized in that** it comprises water or a mixture of water and of hydrophilic organic solvent.

40. Composition according to any one of the preceding claims, **characterized in that** it comprises a fatty phase.

41. Composition according to the preceding claim, **characterized in that** it comprises an ingredient chosen from oils, waxes, pasty fatty substances, gums and their mixtures.

42. Composition according to any one of the preceding claims, **characterized in that** it comprises an organic solvent.

43. Composition according to any one of the preceding claims, **characterized in that** it comprises a film-forming polymer.

44. Composition according to the preceding claim, **characterized in that** the film-forming polymer is chosen from the group formed by vinyl polymers, polyurethanes, polyesters, polyamides, polyureas and cellulose polymers.

45. Composition according to either of Claims 43 and 44, **characterized in that** the film-forming polymer is present in a content, as polymer dry matter, ranging from 0.1% to 60% by weight, with respect to the total weight of the composition.

46. Composition according to any one of the preceding claims, **characterized in that** it comprises a cosmetic ingredient chosen from the group formed by fillers, vitamins, thickening agents, trace elements, softening agents, sequestering agents, fragrances, basifying or acidifying agents, preservatives or their mixtures.

47. Composition according to one of the preceding claims, **characterized in that** it is provided in the form of a nail varnish, mascara, eyeliner, hair composition, product for the lips, foundation, concealer, blusher, eyeshadow, product for the eyebrows or product for making up the body.

48. Composition according to any one of the preceding claims, **characterized in that** it is provided in the form of a nail varnish.

49. Cosmetic method for making up keratinous substances, **characterized in that** a composition according to any one of the preceding claims is applied to the keratinous substances.

50. Cosmetic method for making up keratinous substances comprising the application, to the keratinous substances, of a first layer, also known as base layer, of a first cosmetic composition comprising, in a cosmetically acceptable medium, at least one colouring agent and then the application, over at least a portion of the said first layer, of a second layer of a second cosmetic composition in accordance with the composition as defined according to any one of Claims 1 to 48, the first composition not comprising interference fibres as present in the second composition.

51. Method according to the preceding claim, **characterized in that** the first composition comprises a colouring agent chosen from pigments, pearlescent agents or water-soluble or fat-soluble dyes.

52. Method according to Claim 50 or 51, **characterized in that** the first composition comprises a film-forming polymer.

53. Method according to any one of Claims 50 to 52, **characterized in that** the first composition comprises a cosmetic ingredient chosen from the group formed by fillers, vitamins, thickening agents, trace elements, softening agents, sequestering agents, fragrances, basifying or acidifying agents, preservatives or their mixtures.

54. Method according to any one of Claims 50 to 53, **characterized in that** the first composition is provided in the form of a nail varnish, mascara, eyeliner, hair composition, product for the lips, foundation, concealer, blusher, eyeshadow, product for the eyebrows or product for making up the body.

55. Method according to any one of Claims 50 to 54, **characterized in that** the first composition is provided in the form of a nail varnish.

56. Make-up kit, comprising:

- a first cosmetic composition comprising, in a cosmetically acceptable medium, a first colouring agent, and
- a second cosmetic composition in accordance with the composition as defined according to any one of Claims 1 to 48,
the first composition not comprising interference particles as present in the second composition,
the first and second compositions being packaged in separate containers.

57. Make-up kit according to the preceding claim, **characterized in that** the first composition comprises a colouring agent chosen from pigments, pearlescent agents or water-soluble or fat-soluble dyes.

58. Make-up kit according to Claim 56 or 57,
**characterized in that** the first composition comprises a film-forming polymer.

59. Make-up kit according to any one of Claims 56 to 58, **characterized in that** the first composition comprises a cosmetic ingredient chosen from the group formed by fillers, vitamins, thickeners, trace elements, softening agents, sequestering agents, fragrances, basifying or acidifying agents, preservatives or their mixtures.

60. Make-up kit according to any one of Claims 56 to 59, **characterized in that** the first composition is provided in the form of a nail varnish, mascara, eyeliner, hair composition, product for the lips, foundation, concealer, blusher, eyeshadow, product for the eyebrows or product for making up the body.

61. Make-up kit according to any one of Claims 56 to 60, **characterized in that** the first composition is provided in the form of a nail varnish.

62. Made-up support comprising a make-up capable of being obtained according to the make-up method in accordance with any one of Claims 50 to 55 and applied to the said support, the said support being chosen from false nails, false eyelashes, toupees, wigs, or discs or patches which adhere to the skin or lips.

**Patentansprüche**

1. Zusammensetzung, die in einem physiologisch akzeptablen Medium enthält:

Interferenzfasern aus einem synthetischen Polymer, die einen Farbeffekt ergeben und die einen etwa eiförmigen oder ellipsoidischen Querschnitt und eine Länge L und einen Durchmesser D besitzen, die derart sind, dass L/D im Bereich von 1,2 bis 2500 gewählt ist, sowie
ein zusätzliches Färbemittel,

wobei das Färbemittel in einer solchen Menge vorliegt, dass das Gewichtsverhältnis von Interferenzfasern zu aktivem Material des zusätzlichen Färbemittels 2 bis 500 beträgt.

2. Zusammensetzung nach dem vorhergehenden Anspruch, **dadurch gekennzeichnet, dass** die Interferenzfasern unter Polyesterfasern, Acrylpolymerfasern und Polyamidfasern ausgewählt sind.

3. Zusammensetzung nach einem der vorhergehenden Ansprüche, **dadurch gekennzeichnet, dass** die Interferenzfasern ein Polymer aufweisen, das aus der Gruppe ausgewählt ist, die gebildet wird durch Polyethylenterephthalat, Polyethylennaphthalat, Polycarbonat, Polymethylmethacrylat, Nylon 6, Nylon 6.6, Nylon 6.12, Nylon 11 und Nylon 12.

4. Zusammensetzung nach einem der vorhergehenden Ansprüche, **dadurch gekennzeichnet, dass** die Interferenzfasern Fasern mit Mehrschichtstruktur aus Polymerschichten sind, die abwechselnde Schichten von mindestens einem ersten Polymer und einem zweiten Polymer aufweisen.

5. Zusammensetzung nach dem vorhergehenden Anspruch, **dadurch gekennzeichnet, dass** jede Polymerschicht in einer Ebene (P) liegt, die parallel zur Richtung der Hauptachse der Faser in Richtung ihrer Länge L ist.

6. Zusammensetzung nach Anspruch 4 oder 5, **dadurch gekennzeichnet, dass** der Mehrschichtteil der Faser mindestens 5 individuelle Polymerschichten aufweist.

7. Zusammensetzung nach einem der Ansprüche 4 bis 6, **dadurch gekennzeichnet, dass** der Mehrschichtteil der Faser 5 bis 120 individuelle Polymerschichten aufweist.

8. Zusammensetzung nach einem der Ansprüche 4 bis 7, **dadurch gekennzeichnet, dass** der Mehrschichtteil der Faser 10 bis 50 individuelle Polymerschichten aufweist.

9. Zusammensetzung nach einem der Ansprüche 4 bis 8, **dadurch gekennzeichnet, dass** jede Schicht des ersten Polymers und des zweiten Polymers eine Dicke $d_1$ bzw. $d_2$ aufweist, wobei die Dicken $d_1$ und $d_2$ unabhängig voneinander im Bereich von 0,02 bis 0,3 $\mu$m liegen.

10. Zusammensetzung nach einem der Ansprüche 4 bis 9, **dadurch gekennzeichnet, dass** die in den Fasern vorliegenden Polymeren einen Brechungsindex von 1,3 bis 1,82 aufweisen.

11. Zusammensetzung nach einem der Ansprüche 4 bis 10, **dadurch gekennzeichnet, dass** das erste und das zweite Polymer einen Brechungsindex $n_1$ bzw. $n_2$ aufweisen, wobei das Verhältnis $n_1/n_2$ im Bereich von 1,1 bis 1,4 liegt.

12. Zusammensetzung nach einem der Ansprüche 4 bis 11, **dadurch gekennzeichnet, dass** die Faser mit Mehrschichtstruktur ein solches Reflexionsspektrum aufweist, dass die Halbwertsbreite $\lambda_{L=1/2}$ des Spektrums im Bereich $0 < \lambda_{L=1/2} < 200$ nm liegt.

13. Zusammensetzung nach einem der Ansprüche 4 bis 11, **dadurch gekennzeichnet, dass** das erste Polymer ein Polyamid und das zweite Polymer ein Polyester ist.

14. Zusammensetzung nach einem der vorhergehenden Ansprüche, **dadurch gekennzeichnet, dass** die Fasern oberflächenbehandelt oder mit einer Schutzschicht ummantelt sind.

15. Zusammensetzung nach dem vorhergehenden Anspruch, **dadurch gekennzeichnet, dass** die Schutzschicht ein Polymer aufweist, das aus der Gruppe ausgewählt ist, die gebildet wird durch Polyurethane, Polyethylacrylate und Polyethylmethacrylate.

16. Zusammensetzung nach dem vorhergehenden Anspruch, **dadurch gekennzeichnet, dass** das Polymer der Schutzschicht einen Brechungsindex im Bereich von 1,35 bis 1,55 aufweist.

17. Zusammensetzung nach einem der vorhergehenden Ansprüche, **dadurch gekennzeichnet, dass** die Fasern eine solche Länge L und einen solchen Durchmesser D aufweisen, dass L/D im Bereich von 1,6 bis 150 gewählt ist.

18. Zusammensetzung nach dem vorhergehenden Anspruch, **dadurch gekennzeichnet, dass** die Fasern einen Querschnitt aufweisen, der in einem Kreis mit einem Durchmesser von 2 nm bis 500 $\mu$m liegt.

19. Zusammensetzung nach einem der vorhergehenden Ansprüche, **dadurch gekennzeichnet, dass** die Fasern eine Länge L von 1 $\mu$m bis 10 mm besitzen.

20. Zusammensetzung nach einem der vorhergehenden Ansprüche, **dadurch gekennzeichnet, dass** die Fasern flach sind.

21. Zusammensetzung nach einem der vorhergehenden Ansprüche, **dadurch gekennzeichnet, dass** die Fasern einen Querschnitt besitzen, der eine größere Länge L1 und eine kleinere Länge L2 besitzt, die derart sind, dass L1/L2 größer als 4 ist.

22. Zusammensetzung nach dem vorhergehenden Anspruch, **dadurch gekennzeichnet, dass** das Verhältnis L1/L2 im Bereich von 4 bis 15 liegt.

**23.** Zusammensetzung nach einem der Ansprüche 20 bis 22, **dadurch gekennzeichnet, dass** die flachen Fasern in Form eines Bandes oder in einer bandnudelartigen Form vorliegen.

**24.** Zusammensetzung nach einem der vorhergehenden Ansprüche, **dadurch gekennzeichnet, dass** die Fasern Monofilamentfasern oder Multifilamentfasern sind.

**25.** Zusammensetzung nach einem der vorhergehenden Ansprüche, **dadurch gekennzeichnet, dass** die Fasern längs ihrer Achse der Länge L der Fasern verdrillt sind.

**26.** Zusammensetzung nach einem der vorhergehenden Ansprüche, **dadurch gekennzeichnet, dass** die Fasern einen Titer aufweisen, der im Bereich von 0,15 bis 30 Denier ausgewählt ist.

**27.** Zusammensetzung nach einem der vorhergehenden Ansprüche, **dadurch gekennzeichnet, dass** die Interferenzfasern in einem Mengenanteil von 0,01 bis 50 Gew.-% vorliegen, bezogen auf das Gesamtgewicht der Zusammensetzung.

**28.** Zusammensetzung nach einem der vorhergehenden Ansprüche, **dadurch gekennzeichnet, dass** die Interferenzfasern in einem Mengenanteil von 0,3 bis 20 Gew.-% vorliegen, bezogen auf das Gesamtgewicht der Zusammensetzung.

**29.** Zusammensetzung nach einem der vorhergehenden Ansprüche, **dadurch gekennzeichnet, dass** das zusätzliche Färbemittel aus der Gruppe ausgewählt ist, die gebildet wird durch Pigmente, Perlglanzmittel, wasserlösliche oder öllösliche Farbstoffe und färbende Polymere,

**30.** Zusammensetzung nach einem der vorhergehenden Ansprüche, **dadurch gekennzeichnet, dass** das zusätzliche Färbemittel ein Pigment ist, das ausgewählt ist unter Titandioxid, Zirconiumoxiden, Ceroxiden, Zinkoxiden, Eisenoxiden, Chromoxiden, Manganviolett, Ultramarinblau, Chromhydrat, Eisen(III)-Blau, Aluminiumpulver, Kupferpulver, Ruß, Pigmenten vom Typ D und C und Lacken auf der Basis von Cochenille-Rot, Barium, Strontium, Calcium und Aluminium.

**31.** Zusammensetzung nach Anspruch 29 oder 30, **dadurch gekennzeichnet, dass** die Pigmente in einem Mengenanteil von 0,01 bis 15 Gew.-% vorliegen, bezogen auf das Gewicht der Zusammensetzung.

**32.** Zusammensetzung nach einem der Ansprüche 29 bis 31, **dadurch gekennzeichnet, dass** die Pigmente in einem Mengenanteil von 0,02 bis 5 Gew.-% vorliegen, bezogen auf das Gewicht der Zusammensetzung.

**33.** Zusammensetzung nach Anspruch 29, **dadurch gekennzeichnet, dass** das Färbemittel ein Perlglanzmittel ist, das ausgewählt ist unter titanbeschichtetem Glimmer oder mit Bismutoxychlorid beschichtetem Glimmer, Titanglimmer, der mit Eisenoxiden beschichtet ist, Titanglimmer, der mit Eisen(III)-Blau oder Chromoxid beschichtet ist, Titanglimmer, der mit einem organischen Pigment beschichtet ist, und Perlglanzpigmenten auf der Basis von Bismutoxychlorid.

**34.** Zusammensetzung nach Anspruch 29 oder 30, **dadurch gekennzeichnet, dass** die Perlglanzmittel in einem Mengenanteil von 0,01 bis 25 Gew.-% vorliegen, bezogen auf das Gewicht der Zusammensetzung.

**35.** Zusammensetzung nach einem der Ansprüche 29, 33 oder 34, **dadurch gekennzeichnet, dass** die Perlglanzmittel in einem Mengenanteil von 0,02 bis 5 Gew.-% vorliegen, bezogen auf das Gewicht der Zusammensetzung.

**36.** Zusammensetzung nach einem der vorhergehenden Ansprüche, **dadurch gekennzeichnet, dass** das Färbemittel unter wasserlöslichen oder fettlöslichen Farbstoffen und färbenden Polymeren ausgewählt ist und in einem Mengenanteil an aktivem Material des Farbstoffs von 0,01 bis 6 Gew.-% vorliegt, bezogen auf das Gesamtgewicht der Zusammensetzung.

**37.** Zusammensetzung nach einem der vorhergehenden Ansprüche, **dadurch gekennzeichnet, dass** die Interferenzfasern und das zusätzliche Färbemittel in solchen Mengen vorliegen, dass das Gewichtsverhältnis von Interferenzfasern zu aktivem Material des zusätzlichen Färbemittels im Bereich von 5 bis 500 liegt.

**38.** Zusammensetzung nach einem der vorhergehenden Ansprüche, **dadurch gekennzeichnet, dass** sie ein hydrophiles oder lipophiles kosmetisches Medium aufweist.

39. Zusammensetzung nach einem der vorhergehenden Ansprüche, **dadurch gekennzeichnet, dass** sie Wasser oder ein Gemisch von Wasser mit einem hydrophilen organischen Lösungsmittel aufweist.

40. Zusammensetzung nach einem der vorhergehenden Ansprüche, **dadurch gekennzeichnet, dass** sie eine Fettphase aufweist.

41. Zusammensetzung nach dem vorhergehenden Anspruch, **dadurch gekennzeichnet, dass** die Zusammensetzung einen Bestandteil enthält, der unter Ölen, Wachsen, pastosen Fettstoffen und Gummen sowie Gemischen dieser Stoffe ausgewählt ist.

42. Zusammensetzung nach einem der vorhergehenden Ansprüche, **dadurch gekennzeichnet, dass** sie ein organisches Lösungsmittel enthält.

43. Zusammensetzung nach einem der vorhergehenden Ansprüche, **dadurch gekennzeichnet, dass** sie ein filmbildendes Polymer enthält.

44. Zusammensetzung nach dem vorhergehenden Anspruch, **dadurch gekennzeichnet, dass** das filmbildende Polymer aus der Gruppe ausgewählt ist, die gebildet wird durch Vinylpolymere, Polyurethane, Polyester, Polyamide, Polyharnstoffe und Cellulosepolymere.

45. Zusammensetzung nach Anspruch 43 oder 44, **dadurch gekennzeichnet, dass** das filmbildende Polymer in einem Mengenanteil an Polymer-Feststoffen von 0,1 bis 60 Gew.-% vorliegt, bezogen auf das Gesamtgewicht der Zusammensetzung.

46. Zusammensetzung nach einem der vorhergehenden Ansprüche, **dadurch gekennzeichnet, dass** sie einen kosmetischen Bestandteil enthält, der aus der Gruppe ausgewählt ist, die gebildet wird durch Füllstoffe, Vitamine, Verdickungsmittel, Spurenelemente, reizlindernde Mittel, Sequestrierungsmittel, Parfums, Mittel zum Alkalischmachen, Mittel zum Ansäuern und Konservierungsmittel sowie Gemische dieser Stoffe.

47. Zusammensetzung nach einem der vorhergehenden Ansprüche, **dadurch gekennzeichnet, dass** sie in Form eines Nagellacks, in Form einer Mascara, eines Eyeliners, einer Zusammensetzung für die Haare, eines Produkts für die Lippen, eines Make-ups, eines Produkts gegen Falten, als Rouge oder Lidschatten, als Produkt für die Augenbrauen oder als Produkt zum Schminken des Körpers vorliegt.

48. Zusammensetzung nach einem der vorhergehenden Ansprüche, **dadurch gekennzeichnet, dass** sie in Form eines Nagellacks vorliegt.

49. Kosmetisches Verfahren zum Schminken von Keratinmaterialien, **dadurch gekennzeichnet, dass** eine Zusammensetzung nach einem der vorhergehenden Ansprüche auf die Keratinmaterialien aufgebracht wird.

50. Kosmetisches Verfahren zum Schminken von Keratinmaterialien, das umfasst:

Aufbringen einer ersten Schicht, die auch als Grundschicht bezeichnet wird, einer ersten kosmetischen Zusammensetzung, die in einem kosmetische akzeptablen Medium mindestens ein Färbemittel enthält, auf die Keratinmaterialien und anschließendes Aufbringen einer zweiten Schicht einer zweiten kosmetischen Zusammensetzung gemäß der Zusammensetzung nach einem der Ansprüche 1 bis 48 auf mindestens einen Teil der ersten Schicht, wobei die erste Zusammensetzung keine Interferenzfasern enthält, wie sie in der zweiten Zusammensetzung vorliegen.

51. Verfahren nach dem vorhergehenden Anspruch, **dadurch gekennzeichnet, dass** die erste Zusammensetzung ein Färbemittel enthält, das unter Pigmenten, Perlglanzmitteln und wasserlöslichen oder öllöslichen Farbstoffen ausgewählt ist.

52. Verfahren nach Anspruch 50 oder 51, **dadurch gekennzeichnet, dass** die erste Zusammensetzung ein filmbildendes Polymer enthält.

**53.** Verfahren nach einem der Ansprüche 50 bis 52, **dadurch gekennzeichnet, dass** die erste Zusammensetzung einen kosmetischen Bestandteil enthält, der aus der Gruppe ausgewählt ist, die gebildet wird durch Füllstoffe, Vitamine, Verdickungsmittel, Spurenelemente, reizlindernde Mittel, Sequestrierungsmittel, Parfums, Mittel zum Alkalischmachen, Mittel zum Ansäuern und Konservierungsmittel oder Gemische dieser Stoffe.

**54.** Verfahren nach einem der Ansprüche 50 bis 53, **dadurch gekennzeichnet, dass** die erste Zusammensetzung in Form eines Nagellacks, in Form einer Mascara, eines Eyeliners, einer Zusammensetzung für die Haare, eines Produkts für die Lippen, eines Make-ups, eines Produkts gegen Falten, als Rouge oder Lidschatten, als Produkt für die Augenbrauen oder als Produkt zum Schminken des Körpers vorliegt.

**55.** Verfahren nach einem der Ansprüche 50 bis 54, **dadurch gekennzeichnet, dass** die erste Zusammensetzung in Form eines Nagellacks vorliegt.

**56.** Schmink-Kit, der aufweist:

- eine erste kosmetische Zusammensetzung, die in einem kosmetisch akzeptablen Medium ein erstes Färbemittel enthält, und
- eine zweite kosmetische Zusammensetzung gemäß der Zusammensetzung nach einem der Ansprüche 1 bis 48,

wobei die erste Zusammensetzung keine Interferenzpartikel enthält, wie sie in der zweiten Zusammensetzung vorliegen, und
wobei die erste und die zweite Zusammensetzung in verschiedenen Behältern abgepackt sind.

**57.** Schmink-Kit nach dem vorhergehenden Anspruch, **dadurch gekennzeichnet, dass** die erste Zusammensetzung ein Färbemittel enthält, das unter Pigmenten, Perlglanzmitteln und wasserlöslichen oder öllöslichen Farbstoffen ausgewählt ist.

**58.** Schmink-Kit nach Anspruch 56 oder 57, **dadurch gekennzeichnet, dass** die erste Zusammensetzung ein filmbildendes Polymer enthält.

**59.** Schmink-Kit nach einem der Ansprüche 56 bis 58, **dadurch gekennzeichnet, dass** die erste Zusammensetzung einen kosmetischen Bestandteil enthält, der aus der Gruppe ausgewählt ist, die gebildet wird durch Füllstoffe, Vitamine, Verdickungsmittel, Spurenelemente, reizlindernde Mittel, Sequestrierungsmittel, Parfums, Mittel zum Alkalischmachen, Mittel zum Ansäuern und Konservierungsstoffe oder Gemische dieser Stoffe.

**60.** Schmink-Kit nach einem der Ansprüche 56 bis 59, **dadurch gekennzeichnet, dass** die erste Zusammensetzung in Form eines Nagellacks, in Form einer Mascara, eines Eyeliners, einer Zusammensetzung für die Haare, eines Produkts für die Lippen, eines Make-ups, eines Produkts gegen Falten, als Rouge oder Lidschatten, als Produkt für die Augenbrauen oder als Produkt zum Schminken des Körpers vorliegt.

**61.** Schmink-Kit nach einem der Ansprüche 56 bis 60, **dadurch gekennzeichnet, dass** die erste Zusammensetzung in Form eines Nagellacks vorliegt.

**62.** Geschminkter Träger, der eine Schminke aufweist, die erhältlich ist nach dem Verfahren nach einem der Ansprüche 50 bis 55 und auf den Träger aufgebracht ist, wobei der Träger ausgewählt ist unter falschen Nägeln, falschen Wimpern, Haarteilen, Perücken und Zweitfrisuren, Pastillen oder Patches, die auf der Haut oder den Lippen haften.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 571158714 A
- US 5498407 A
- EP 0106762 A
- FR 1529329 A
- JP 7196440 A
- EP 921217 A
- JP 2003019131 A
- EP 686858 A
- US 5472798 A
- US 3438796 A
- EP 227423 A
- US 5135812 A
- EP 170439 A
- EP 341002 A
- US 4930866 A
- US 5641719 A
- EP 472371 A
- EP 395410 A
- EP 753545 A
- EP 768343 A
- EP 571836 A
- EP 708154 A
- EP 579091 A
- US 5411586 A
- US 5364467 A
- WO 9739066 A
- DE 4225031 A
- WO 9517479 A
- DE 19614637 A
- WO 9844902 A
- WO 9629046 A
- WO 9201022 A
- WO 9007558 A
- BE 609054 A
- US 4267306 A
- US 4359570 A
- US 4403092 A
- US 4617373 A
- US 4080355 A
- US 4740581 A
- US 4116923 A
- US 4745173 A
- US 4804719 A
- US 5194463 A
- US 5804719 A
- WO 9207913 A
- US 5032670 A
- US 4999418 A
- US 5106942 A
- US 5030708 A
- US 5102980 A
- US 5043376 A
- WO 9724102 A